# EUROPÄISCHE PATENTSCHRIFT

(11) **EP 2 633 841 B1**
(45) Veröffentlichungstag und Bekanntmachung des Hinweises auf die Patenterteilung: **16.08.2017**
(21) Anmeldenummer: 12001739.7
(22) Anmeldetag: 14.03.2012
(51) Int. Cl.: A61F 9/008, A61F 9/009

(54) **Vorrichtung zur Behandlung von Augengewebe mittels gepulster Laserstrahlen**
Device for treating eye tissue using pulsed laser beams
Dispositif de traitement de tissus oculaires par rayon laser à impulsion

(30) Priorität: 28.02.2012 US 201261604149 P
(43) Veröffentlichungstag der Anmeldung: 04.09.2013
(73) Patentinhaber: Ziemer Ophthalmic Systems AG, 2562 Port (CH)
(72) Erfinder: Rathjen, Christian, 28197 Bremen (DE)
(74) Vertreter: Rentsch Partner AG

(56) Entgegenhaltungen:
- WO-A1-2011/011202
- US-A1- 2011 028 948
- US-A1- 2011 172 649

## Beschreibung

### Technisches Gebiet

Die vorliegende Erfindung betrifft eine ophthalmologische Vorrichtung zur Behandlung von Augengewebe mittels gepulster Laserstrahlen. Die vorliegende Erfindung betrifft insbesondere eine ophthalmologische Vorrichtung zur Behandlung von Augengewebe mittels gepulster Laserstrahlen, welche ein Lasersystem umfasst, das eingerichtet ist, in einem ersten Betriebsmodus gepulste Laserstrahlen mit einer Wellenlänge im NIR-Infrarotbereich und in einem zweiten Betriebsmodus gepulste Laserstrahlen mit einer Wellenlänge im UVA-Ultraviolettbereich zu erzeugen.

### Stand der Technik

Fehlsichtigkeiten wie Myopie (Kurzsichtigkeit), Hyperopie (Weitsichtigkeit oder Übersichtigkeit) oder Astigmatismus (Stabsichtigkeit) können heute durch refraktiv-chirurgische Behandlung dauerhaft korrigiert werden. Refraktiv-chirurgische Behandlungen sind chirurgische Eingriffe am Auge, die die optische Brechkraft des Auges ändern mit dem Ziel, diese einem gewünschten Wert möglichst gut anzunähern. Eines der bedeutendsten Verfahren in der refraktiven Chirurgie ist die so genannte Laser in-situ Keratomileusis (LASIK), bei der das Innere der Hornhaut mit einem Excimerlaser abgetragen wird, nachdem zuvor ein Hornhautlappen teilweise abgetrennt und weggeklappt wird. Solche Hornhautlappen werden mit mechanischen Mikrokeratomen oder mittels stark fokussierter Femtosekundenlaserpulsen geschnitten. Geeignete Femtosekundenlasersysteme erzeugen Laserpulse mit Pulsbreiten von typisch 100fs bis 1000fs (1fs=10-15s).

In der Patentanmeldung WO 03/057100 wird eine Vorrichtung für die refraktive Laserchirurgie beschrieben, welche ein Lasersystem zur Erzeugung eines gepulsten Laserstrahls mit Femtosekundenlaserpulsen und ein weiteres Lasersystem zur Erzeugung eines Laserstrahls im ultravioletten (UV) Bereich umfasst. Vorzugsweise werden beide Lasersysteme durch eine gemeinsame Pumplaserquelle im Infrarotbereich gespeist, wobei die UV Laserstrahlen mittels Frequenzvervielfachung aus dem Infrarotlicht erzeugt werden. In einer Ausführungsvariante wird zur Erzeugung des UV Laserstrahls ein Excimerlaser verwendet. Die Lichtstrahlen beider Lasersysteme werden einem gemeinsamen Scanner zugeführt, der sowohl zur Ablenkung der Femtosekundenlaserpulse beim Schneiden des Hornhautlappens als auch zur Ablenkung des UV Laserstrahls bei der refraktiven Korrektur der Hornhaut durch Oberflächenabtrag verwendet wird.

Die Patentanmeldung US 2011/028948 beschreibt ein ophthalmologisches Laserbehandlungssystem, das einen Scanner und mehrere dem Projektionsobjektiv vorgeschaltete Beam Expander umfasst.

Die Patentanmeldung US 2011/172649 beschreibt ein ophthalmologisches Laserbehandlungssystem für intraokulare Ziele, das eine Laserquelle im Wellenlängenbereich von 320nm bis 430nm verwendet. Das Laserbehandlungssystem gemäss US 2011/172649 wird für Schnitte in der Hornhaut, in einer implantierten intraokularen Linse und im Linsensack eingesetzt.

Die Patentanmeldung WO 2011/011202 beschreibt ein ophthalmologisches Laserbehandlungssystem, das einen Laser zur Erzeugung eines Laserstrahls umfasst, wobei Wellenlängen von etwa 300nm bis 2500nm verwendet werden können. Das Laserbehandlungssystem umfasst eine Laseroptik mit einem Beam Expander Teleskop, einem z-Fokus Mechanismus, einer Fokussieroptik und einem x-y Scanner. Um den Laserstrahl den Augenstrukturen einschliesslich Hornhaut und Linse zuzuführen, ist die Laseroptik gemäss WO 2011/011202 eingerichtet, eine Serie von Pulsen in einem präzisen Muster in x, y und z Richtung zu liefern.

### Darstellung der Erfindung

Es ist eine Aufgabe der vorliegenden Erfindung eine verbesserte ophthalmologische Vorrichtung zur Behandlung von Augengewebe mittels gepulster Laserstrahlen vorzuschlagen, welche ein Lasersystem umfasst, das eingerichtet ist, in verschiedenen Betriebsmodi gepulste Laserstrahlen mit verschiedenen Wellenlängen zu erzeugen, insbesondere in einem ersten Betriebsmodus im NIR-Infrarotbereich und in einem zweiten Betriebsmodus im UVA-Ultraviolettbereich.

Gemäss der vorliegenden Erfindung werden diese Ziele durch die Merkmale der unabhängigen Ansprüche erreicht. Weitere vorteilhafte Ausführungsformen gehen ausserdem aus den abhängigen Ansprüchen und der Beschreibung hervor.

Eine ophthalmologische Vorrichtung zur Behandlung von Augengewebe, insbesondere zur Materialbearbeitung im Augengewebe, mittels gepulster Laserstrahlen umfasst ein Lasersystem, das eingerichtet ist, in einem ersten Betriebsmodus gepulste Laserstrahlen mit einer Wellenlänge im NIR-Infrarotbereich zu erzeugen und in einem zweiten Betriebsmodus gepulste Laserstrahlen mit einer Wellenlänge im UVA-Ultraviolettbereich zu erzeugen.

Die oben genannten Ziele werden durch die vorliegende Erfindung insbesondere dadurch erreicht, dass die ophthalmologische Vorrichtung ein Fokussiersystem mit einer Projektionsoptik umfasst, das eingerichtet ist, im ersten Betriebsmodus die gepulsten Laserstrahlen im NIR-Infrarotbereich mit einer ersten Zoomfunktion zum Auflösen von Augengewebe auf eine erste Spotgrösse fokussiert in die Augenlinse zu projizieren, und im zweiten Betriebsmodus die gepulsten Laserstrahlen im UVA-Ultraviolettbereich mit einer von der ersten Zoomfunktion verschiedenen, zweiten Zoomfunktion zum Erzeugen von Gewebeschnitten auf eine zweite Spotgrösse, die wesentlich kleiner als die erste Spotgrösse ist, fokussiert in die Augenhornhaut zu projizieren.

Das Fokussiersystem ist insbesondere eingerichtet, im ersten Betriebsmodus die gepulsten Laserstrahlen im NIR-Infrarotbereich mit der ersten Zoomfunktion auf eine in der Augenlinse liegende Abbildungsfläche zu projizieren und auf die erste Spotgrösse zu fokussieren, und im zweiten Betriebsmodus die gepulsten Laserstrahlen im UVA-Ultraviolettbereich mit der zweiten Zoomfunktion auf eine in der Augenhornhaut liegende Abbildungsfläche zu projizieren und auf die zweite Spotgrösse zu fokussieren.

In einer Ausführungsvariante umfasst das Fokussiersystem zwei Optiksysteme, welche durch die Zoomfunktionen einstellbar sind, die gepulsten Laserstrahlen auf eine Abbildungsfläche zu projizieren und auf der Abbildungsfläche auf eine Spotgrösse zu fokussieren. Die Optiksysteme sind somit durch die Zoomfunktionen einstellbar, die gepulsten Laserstrahlen auf eine durch die betreffende Zoomfunktion bestimmte Abbildungsfläche zu projizieren und auf dieser Abbildungsfläche auf eine durch die betreffende Zoomfunktion bestimmte Spotgrösse zu fokussieren.

In einer Ausführungsvariante umfasst das Fokussiersystem ein durch die Zoomfunktionen steuerbares Antriebssystem zum individuellen Einstellen der Optiksysteme. Das Fokussiersystem ermöglicht somit eine durch die Zoomfunktonen gesteuerte automatische Einstellung der Optiksysteme, derart, dass die gepulsten Laserstrahlen auf eine durch die betreffende Zoomfunktion bestimmte Abbildungsfläche projiziert werden und auf dieser Abbildungsfläche auf eine durch die betreffende Zoomfunktion bestimmte Spotgrösse fokussiert werden.

In einer weiteren Ausführungsvariante ist das Fokussiersystem eingerichtet, im ersten Betriebsmodus mit der ersten Zoomfunktion die Optiksysteme einzustellen, die gepulsten Laserstrahlen im NIR-Infrarotbereich auf die in der Augenlinse liegende Abbildungsfläche zu projizieren und auf der in der Augenlinse liegenden Abbildungsfläche auf die erste Spotgrösse zu fokussieren, und im zweiten Betriebsmodus mit der zweiten Zoomfunktion die Optiksysteme einzustellen, die gepulsten Laserstrahlen im UVA-Ultraviolettbereich auf die in der Augenhornhaut liegende Abbildungsfläche zu projizieren und auf der in der Augenhornhaut liegenden Abbildungsfläche auf die zweite Spotgrösse zu fokussieren.

In verschiedenen Ausführungsvarianten umfasst das Fokussiersystem in den Strahlengang einschiebbare optische Linsen, deformierbare Spiegel, mechanisch ausgeführte Zoomkurven zur Ausführung der Zoomfunktionen und/oder Zoomfunktionen mit digitalisierten Zoomkurven für ein Steuersystem.

In einer Ausführungsvariante ist das Lasersystem eingerichtet, im ersten Betriebsmodus gepulste Laserstrahlen mit einer Wellenlänge im IR-A-Infrarotbereich zu erzeugen und in einem dritten Betriebsmodus gepulste Laserstrahlen mit einer Wellenlänge im IR-B-Infrarotbereich zu erzeugen, und das Fokussiersystem ist eingerichtet, im dritten Betriebsmodus die gepulsten Laserstrahlen im IR-B-Infrarotbereich zum Auflösen von Augengewebe fokussiert in die Augenlederhaut oder getrübte Hornhaut zu projizieren.

In einer weiteren Ausführungsvariante ist das Fokussiersystem eingerichtet, im dritten Betriebsmodus die gepulsten Laserstrahlen im IR-B-Infrarotbereich mit einer dritten Zoomfunktion auf eine in der Augenlederhaut oder einer getrübten Hornhaut liegende Abbildungsfläche zu projizieren und auf eine dritte Spotgrösse zu fokussieren.

In einem weiteren Aspekt betrifft die vorliegende Erfindung eine ophthalmologische Vorrichtung zur Behandlung von Augengewebe mittels gepulster Laserstrahlen, welche ein Lasersystem umfasst, das eingerichtet ist, in verschiedenen Betriebsmodi gepulste Laserstrahlen mit unterschiedlichen Wellenlängen zu erzeugen, und welche ein Fokussiersystem mit einer Projektionsoptik umfasst, das eingerichtet ist, in den verschiedenen Betriebsmodi die gepulsten Laserstrahlen mit den verschiedenen Wellenlängen jeweils mit einer der betreffenden Wellenlänge zugeordneten unterschiedlichen Zoomfunktion fokussiert in das Augengewebe zu projizieren.

In einer Ausführungsvariante ist das Fokussiersystem eingerichtet, die gepulsten Laserstrahlen in verschiedenen Betriebsmodi mit einem unterschiedlichen, durch eine dem betreffenden Betriebsmodus zugeordnete Zoomfunktion bestimmten Intensitätsprofil fokussiert auf die Abbildungsfläche zu projizieren.

In einer Ausführungsvariante umfasst die ophthalmologische Vorrichtung ein Steuersystem, das eingerichtet ist, die ophthalmologische Vorrichtung gemäss verschiedenen Betriebsmodi zu steuern, wobei die Betriebsmodi mindestens eines umfassen aus:
- einen ersten Betriebsmodus zum Auflösen von Augengewebe der Augenlinse durch Fokussieren von gepulsten Laserstrahlen im IR-A-Infrarotbereich mit einer ersten Zoomfunktion,
- einen zweiten Betriebsmodus zum Erzeugen von horizontalen Gewebeschnitten in der Hornhaut durch Fokussieren der gepulsten Laserstrahlen im UVA-Ultraviolettbereich mit einer zweiten Zoomfunktion,
- einen dritten Betriebsmodus zum Auflösen von Augengewebe der Augenlederhaut oder getrübten Hornhaut durch Fokussieren von gepulsten Laserstrahlen im IR-B-Infrarotbereich mit einer dritten Zoomfunktion,
- einen vierten Betriebsmodus zum Erzeugen von Gewebeschnitten im Linsensack der Augenlinse durch Fokussieren der gepulsten Laserstrahlen im UVA-Ultraviolettbereich mit einer vierten Zoomfunktion, und
- einen fünften Betriebsmodus zum Erzeugen von vertikalen Gewebeschnitten in der Hornhaut durch Fokussieren von gepulsten Laserstrahlen im IR-A-Infrarotbereich mit einer fünften Zoomfunktion.

Vorzugsweise ist die Projektionsoptik für die gepulsten Laserstrahlen im UVA-Ultraviolettbereich transparent ausgeführt und weist eine tiefe numerische Apertur NA, insbesondere eine numerische Apertur NA<0.5, auf, beispielsweise eine numerische Apertur NA<0.3 oder NA<0.2.

In einer Ausführungsvariante weisen die in der Projektionsoptik verwendeten Gläser einen Brechungsindex n<1.65 auf. Die in der Projektionsoptik verwendeten Gläser sind vorzugsweise aus Quarzglas. Die Erfindung wird durch die Ansprüche definiert.

### Kurze Beschreibung der Zeichnungen

Nachfolgend wird eine Ausführung der vorliegenden Erfindung anhand eines Beispiels beschrieben. Das Beispiel der Ausführung wird durch die folgenden beigelegten Figuren illustriert:
- Figur 1:: zeigt ein Blockdiagram, welches schematisch eine ophthalmologische Vorrichtung zur Behandlung von Augengewebe mittels gepulster Laserstrahlen illustriert, wobei für die fokussierte Projektion der gepulsten Laserstrahle auf Abbildungsflächen in der Hornhaut, der Augenlinse und der Lederhaut unterschiedliche Spotgrössen bestimmt werden.
- Figur 2:: zeigt ein Blockdiagram, welches schematisch ein Lasersystem illustriert, das eine Laserquelle zur Erzeugung eines gepulsten Laserstrahls mit einer Anfangswellenlänge und einen Frequenzwandler umfasst zur Erzeugung eines gepulsten Laserstrahls mit einer Zielwellenlänge aus dem Laserstrahl mit der Anfangswellenlänge.
- Figur 3:: zeigt ein Blockdiagram, welches schematisch ein Lasersystem illustriert, das mehrere Laserquellen umfasst, die jeweils zur Erzeugung von gepulsten Laserstrahlen einer unterschiedlichen Wellenlänge eingerichtet sind.
- Figur 4:: zeigt ein Blockdiagram, welches schematisch eine ophthalmologische Vorrichtung zur Behandlung von Augengewebe mittels gepulster Laserstrahlen illustriert, in welcher das Lasersystem in einer Basisstation angeordnet ist, und in welcher die Projektionsoptik in einem Applikationskopf angeordnet ist, der mit einem Tragarm an der Basisstation angebracht ist.
- Figur 5:: zeigt eine schematische Darstellung von Profilen eines gepulsten Laserstrahls in verschiedenen Zuständen, in welchen der gepulste Laserstrahl auf verschiedene Abbildungsflächen projiziert und jeweils mit einer unterschiedlichen definierten Spotgrösse fokussiert wird.
- Figur 6:: zeigt eine schematische Darstellung von Profilen gepulster Laserstrahlen mit unterschiedlicher Wellenlänge, die auf eine Abbildungsfläche projiziert und mit einer unterschiedlichen Spotgrösse fokussiert werden.
- Figur 7:: zeigt in der Aufsicht (oben) und im Querschnitt (unten) schematisch eine Fragmentierung der Augenlinse mit vertikalen, parallel zur Projektionsrichtung des gepulsten Laserstrahls verlaufenden Gewebeschnitten.
- Figur 8:: zeigt in der Aufsicht (oben) und im Querschnitt (unten) schematisch eine Fragmentierung der Augenlinse mit vertikalen und horizontalen Gewebeschnitten, die parallel respektive normal zur Projektionsrichtung des gepulsten Laserstrahls verlaufen.
- Figur 9:: zeigt schematisch im Querschnitt einen in der Hornhaut geschnittenen Gewebelappen ("flap"), der in einem Restbereich mit dem Auge verbunden bleibt.
- Figur 10:: zeigt schematisch im Querschnitt einen von der Hornhaut weggeschnittenen Gewebeteil für eine teilweise Hornhauttransplantation.
- Figur 11:: zeigt schematisch im Querschnitt eine vom Auge weggeschnittene Hornhaut für eine vollständige Hornhauttransplantation.
- Figur 12:: zeigt schematisch in der Aufsicht die Stützung eines Keratokonus mittels einer mechanischen Stützeinlage, die in eine in die Hornhaut geschnittene gekrümmte Tasche eingeführt ist.
- Figur 13:: zeigt schematisch in der Aufsicht eine in die Hornhaut geschnittene gekrümmte Tasche zur Aufnahme eines Implantats zum Glattziehen einer durch ein Keratokonus erzeugten Ausbeulung der Hornhaut.
- Figur 14:: zeigt schematisch in der Aufsicht mehrere sternförmig angeordnete, in die Hornhaut geschnittene Taschen für die Korrektur eines Keratokonus.
- Figur 15:: zeigt schematisch in der Aufsicht zwei mit einem Abstand übereinanderliegend und sich kreuzend in die Hornhaut geschnittene Taschen zum Einführen von Stützeinlagen zur Stützung eines Keratokonus.

### Wege zur Ausführung der Erfindung

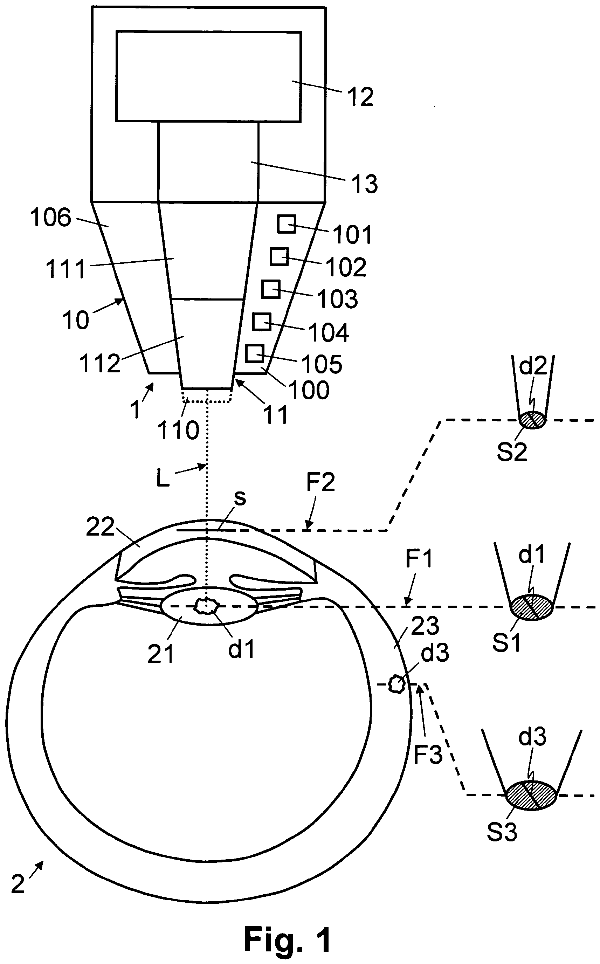

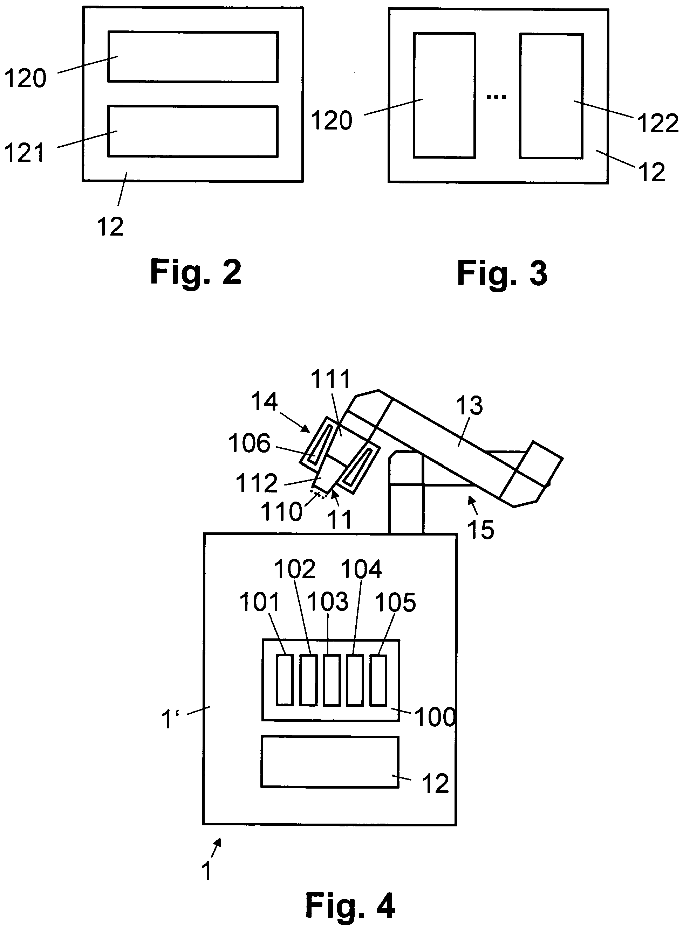

In den Figuren 1 und 4 bezieht sich das Bezugszeichen 1 auf eine ophthalmologische Vorrichtung zur Behandlung von Augengewebe, insbesondere zur Materialbearbeitung im Augengewebe, mittels gepulster Laserstrahlen L. Wie in den Figuren 1 und 4 schematisch dargestellt ist, umfasst die ophthalmologische Vorrichtung 1 ein Lasersystem 12, das eingerichtet ist, in verschiedenen Betriebsmodi gepulste Laserstrahlen L, insbesondere Femtosekundenlaserpule (1fs=10⁻¹⁵s), mit unterschiedlichen Wellenlängen zu erzeugen. Die ophthalmologische Vorrichtung 1 umfasst überdies ein Fokussiersystem 10, das eingerichtet ist, in den verschiedenen Betriebsmodi die gepulsten Laserstrahlen L mit den verschiedenen Wellenlängen jeweils mit einer der betreffenden Wellenlänge zugeordneten unterschiedlichen Zoomfunktion fokussiert in das Augengewebe zu projizieren, auf verschiedene Abbildungsflächen mit verschiedenen Spotgrössen und/oder Intensitätsprofilen, wie später detaillierter beschrieben wird.

Das Lasersystem 12 ist insbesondere eingerichtet, gepulste Laserstrahlen L im NIR-Infrarotbereich (naher Infrarotbereich), z.B. wählbar im kurzwelligeren IR-A-Infrarotbereich, insbesondere im Bereich zwischen 1000nm und 1100nm, oder im langwelligeren IR-B-Infrarotbereich, insbesondere im Bereich zwischen 1600nm und 1700nm, und im UVA-Ultraviolettbereich (naher Ultraviolettbereich), insbesondere im Bereich zwischen 300nm und 400nm, zu erzeugen.

In der Ausführungsvariante gemäss Figur 2 umfasst das Lasersystem 12 eine Laserquelle 120, die eingerichtet ist, gepulste Laserstrahlen L mit einer Anfangswellenlänge respektive Anfangsfrequenz zu erzeugen, und einen Frequenzwandler 121, der eingerichtet ist, durch Frequenzwandlung aus den gepulsten Laserstrahlen L der Anfangswellenlänge respektive Anfangsfrequenz gepulste Laserstrahlen L in der Zielfrequenz respektive Zielwellenlänge zu erzeugen, beispielsweise durch Frequenzvervielfachung aus Laserstrahlen im NIR-Infrarotbereich Laserstrahlen im UVA-Ultraviolettbereich, oder umgekehrt durch Frequenzteilung aus Laserstrahlen im UVA-Ultraviolettbereich Laserstrahlen im NIR-Infrarotbereich.

In der Ausführungsvariante gemäss Figur 3 umfasst das Lasersystem 12 mehrere separate Laserquellen 120, 122, die jeweils zur Erzeugung von gepulsten Laserstrahlen L einer unterschiedlichen Wellenlänge eingerichtet sind, beispielsweise eine erste Laserquelle 120 im IR-A-Infrarotbereich, eine zweite Laserquelle im IR-B-Infrarotbereich, und eine dritte Laserquelle 122 im UVA-Ultraviolettbereich, und die selektiv aktivierbar sind.

Wie in den Figuren 1 und 4 schematisch dargestellt ist, umfasst die ophthalmologische Vorrichtung 1 überdies ein optisches Übertragungssystem 13 zur Übertragung des gepulsten Laserstrahls respektive der Laserpulse vom Lasersystem 12 an eine Projektionsoptik 11, welche eingerichtet ist, den Laserstrahl L respektive dessen Laserpulse fokussiert ins Augengewebe zu projizieren. Wie in den Figuren 1 und 4 schematisch dargestellt ist, umfasst die Projektionsoptik 11 zwei individuell einstellbare Optiksysteme 111, 112, beispielsweise zwei Linsengruppen mit jeweils einer oder mehreren bewegbaren Linsen, und/oder ein oder mehrere deformierbare Spiegel/Linsen und einschiebbare Korrektur-Elemente. Die beiden Optiksysteme 111, 112 sind mit einem Antriebssystem 106 gekoppelt, welches einen oder mehrere elektrische Motoren umfasst und eingerichtet ist, die Optiksysteme 111, 112 individuell einzustellen, beispielsweise durch Verschieben von Linsen in der Projektionsrichtung (Fokustiefe) und/oder normal zur Projektionsrichtung (in den/aus dem Strahlengang). Die Projektionsoptik 11 ist für gepulste Laserstrahlen L im UVA-Ultraviolettbereich transparent ausgeführt. Die in der Projektionsoptik 11 verwendeten Gläser weisen einen Brechungsindex n< 1.65 auf. Die in der Projektionsoptik 11 verwendeten Gläser sind aus Quarzglas ausgeführt. Die Projektionsoptik 11 weist eine tiefe numerische Apertur NA auf, insbesondere eine numerische Apertur NA<0.5, beispielsweise eine numerische Apertur NA<0.3 oder NA<0.2. Das optische Übertragungssystem 13 umfasst zudem ein Scannersystem zur lateralen Strahlablenkung in einer oder mehreren Scannrichtungen, auf welches nicht näher eingegangen wird.

Wie in den Figuren 1 und 4 schematisch dargestellt ist, umfasst die ophthalmologische Vorrichtung 1 zudem ein Steuersystem 100. Das Steuersystem 100 umfasst einen oder mehrere mit Programm- und Datenspeicher gekoppelte Prozessoren oder andere programmierte Logikeinheiten und/oder Elektronikeinheiten zur Steuerung der ophthalmologischen Vorrichtung 1. Das Steuersystem 100 umfasst insbesondere mehrere verschiedene Zoomfunktionen 101, 102, 103, 104, 105, die eingerichtet sind, Zoom-Steuersignale zur Steuerung des Antriebssystems 106 respektive der Optiksysteme 111, 112 zu erzeugen und zu übermitteln. Wie in der Figur 1 illustriert ist, bilden das Steuersystem 100 und die Zoomfunktionen 101, 102, 103, 104, 105 zusammen mit den Optiksystemen 111, 112 der Projektionsoptik 11 und dem Antriebssystem 106 ein Fokussiersystem 10, das durch die Zoomfunktionen 101, 102, 103, 104, 105 gesteuert wird, wie nachfolgend detailliert beschrieben wird.

In der Ausführungsvariante gemäss Figur 4 umfasst die ophthalmologische Vorrichtung 1 eine Basisstation 1', in welcher das Lasersystem 12 und das Steuersystem 100 angeordnet sind. In der Ausführungsvariante der Figur 4 sind die Projektionsoptik 11 und das Antriebssystem 106 in einem Applikationskopf 14 angeordnet, der manuell auf das Auge 2 aufsetzbar ist und das Auge 2 beispielsweise mit einem transparenten Kontaktkörper 110 berührt, der fest oder entfernbar mit dem Applikationskopf 14 gekoppelt ist. Der Applikationskopf 14 ist mit einem Tragarm 15 an der Basisstation 1' angebracht und umfasst das optische Übertragungssystem 13, welches das Lasersystem 12 optisch mit der Projektionsoptik 11 verbindet. Der Tragarm ist beispielsweise als in sich starrer Tragarm oder als beweglicher Gelenkarm ausgeführt, zum Beispiel ein beweglicher Gelenkarm gemäss EP 1 731 120.

Die verschiedenen selektierbaren Zoomfunktionen 101, 102, 103, 104, 105 des Steuersystems 100 sind jeweils eingerichtet, das Fokussiersystem 10 so zu steuern, dass der gepulste Laserstrahl L (respektive seine Laserpulse) auf eine spezifisch für die betreffende Zoomfunktion 101, 102, 103, 104, 105 definierte Abbildungsfläche F, F1, F2, F3 projiziert wird, wodurch die Behandlungstiefe bestimmt wird, und dass der gepulste Laserstrahl L (respektive seine Laserpulse) auf dieser Abbildungsfläche F mit spezifisch für die betreffende Zoomfunktion 101, 102, 103, 104, 105 definierter Spotgrösse d und definiertem Intensitätsprofil im Querschnitt des Laserstrahls L fokussiert wird. Die Zoomfunktionen 101, 102, 103, 104, 105 sind eingerichtet, das Antriebssystem 106 zum parallelen (gleichzeitig) oder sequentiellen (hintereinander) Einstellen der Optiksysteme 111, 112 anzusteuern, durch Übermittlung der entsprechenden Zoom-Steuersignalen an das Antriebssystem 106 des Fokussiersystems 10. Der Fachmann wird verstehen, dass die Zoomfunktionen 101, 102, 103, 104, 105 in einer alternativen Ausführungsvariante als mechanische Zoomkurven ausgeführt sein können. Die Zoomfunktionen 101, 102, 103, 104, 105 ermöglichen somit, den gepulsten Laserstrahl L respektive dessen Laserpulse auf die Abbildungsfläche F, F1, F2, F3 in der gewünschten Behandlungstiefe zu projizieren und auf dieser Abbildungsfläche F, F1, F2, F3 den Spot auf die gewünschte Spotgrösse d, d 1, d2, d3 mit dem gewünschten Intensitätsprofil (nach-)zufokussieren.

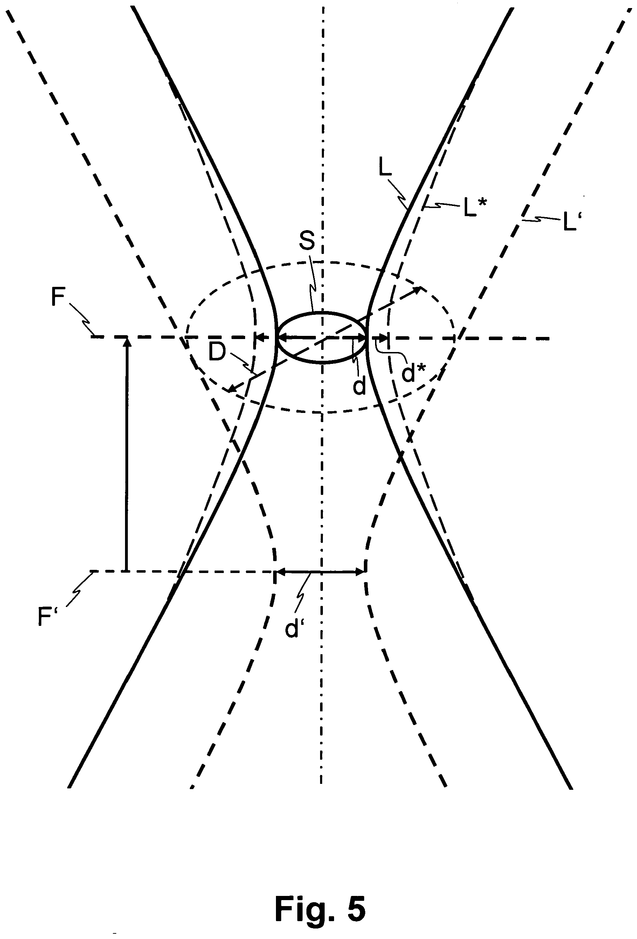

In der Figur 5 beziehen sich die Bezugszeichen L', L*, L auf gepulste Laserstrahlen, die mit ihren Laserstrahlprofilen, z.B. Gauss'sche Laserstrahlprofile, bei verschiedenen Zuständen des Fokussiersystems 10 dargestellt sind, wobei die Zentrumsachse des Laserstrahls L', L*, L respektive die Projektionsrichtung mit dem Bezugszeichen z bezeichnet ist.

Das Bezugszeichen L' bezieht sich auf den gepulsten Laserstrahl in einem ersten Zustand, in welchem die schmalste Strahltaille des Strahlprofils mit einer einen Durchmesser d' aufweisenden Spotgrösse auf die Abbildungsfläche F' projiziert wird. In diesem ersten Zustand wird der gepulste Laserstrahl L' somit auf die Projektions- oder Abbildungsfläche F' projiziert. In diesem ersten Zustand hat das Strahlprofil auf der oberen Abbildungsfläche F einen Durchmesser D, welcher bedeutend grösser ist als sein Durchmesser d' auf der Abbildungsfläche F'.

Das Bezugszeichen L* bezieht sich auf den gepulsten Laserstrahl im Zustand, in welchem der Fokus des gepulsten Laserstrahls von der Abbildungsfläche F' auf die Abbildungsfläche F verschoben wurde. In diesem zweiten Zustand wird der gepulste Laserstrahl L*, wie in der Figur 5 ersichtlich ist, mit einer breiteren Strahltaille d*>d' auf die Projektions- oder Abbildungsfläche F projiziert.

Das Bezugszeichen L bezieht sich auf den gepulsten Laserstrahl in einem dritten Zustand, in welchem der schmalste Querschnitt des Strahlprofils (Spot S) mit einer einen Durchmesser d=d' aufweisenden Spotgrösse auf die Abbildungsfläche F projiziert wird, wobei dieser Durchmesser d kleiner ist als der Durchmesser d* im zweiten Zustand. Im dritten Zustand wird der gepulste Laserstrahl L somit als Spot S, mit einer einen Durchmesser d=d' aufweisenden Spotgrösse, von der Spotgrösse mit Durchmesser d* auf die Spotgrösse mit Durchmesser d=d' *nachfokussiert* auf die Abbildungsfläche F projiziert.

Die Zoomfunktionen 101, 102, 103, 104, 105 respektive Zoom-Steuersignale steuern das Antriebssystem 106 respektive die Optiksysteme 111, 112 so, dass das Fokussiersystem 10 ausgehend von einem Grundzustand, z.B. der oben beschriebene erste oder zweite Zustand, in einen Zielstand geführt wird, z.B. der oben beschriebene dritte Zustand, um den gepulsten Laserstrahl L respektive dessen Laserpulse auf die durch die betreffende Zoomfunktion 101, 102, 103, 104, 105 definierte Abbildungsfläche F, F1, F2, F3 an der definierten Behandlungstiefe zu projizieren und auf dieser Abbildungsfläche F, F1, F2, F3 mit einer durch die betreffende Zoomfunktion bestimmten Spotgrösse d, d 1, d2, d3 und einem durch die betreffende Zoomfunktion bestimmten (radialen) Intensitätsprofil zu fokussieren.

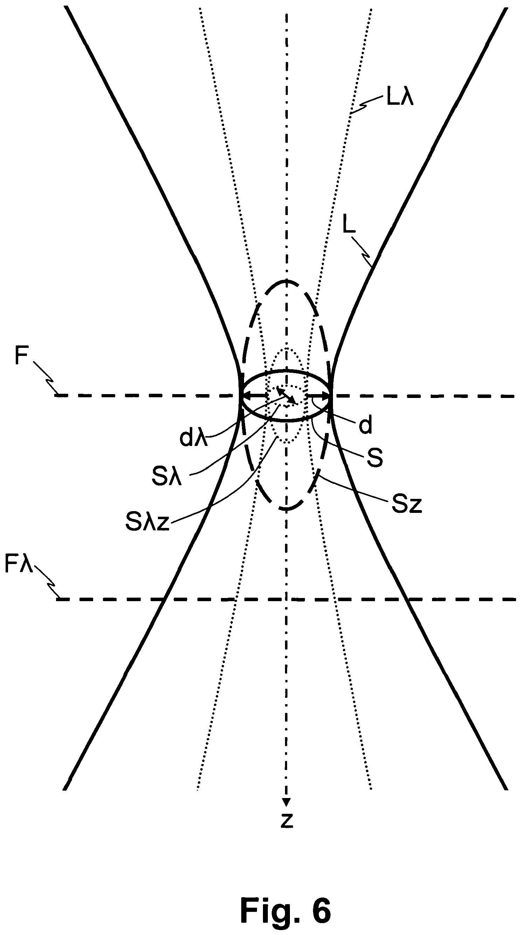

Die Figur 6 illustriert schematisch die Laserstrahlprofile von gepulsten Laserstrahlen L, Lλ unterschiedlicher Wellenlänge bei (nach-)fokussierter Projektion auf die Abbildungsfläche F, wobei der gepulste Laserstrahl L langwelliger ist, z.B. im NIR-Infrarotbereich (naher Infrarotbereich), als der gepulste Laserstrahl Lλ, z.B. im UVA-Ultraviolettbereich (naher Ultraviolettbereich). Bei einer Änderung der Wellenlänge verschiebt sich der Fokus in Projektionsrichtung z, d.h. bei unveränderter Einstellung der Projektionsoptik sind die Abbildungsflächen F, Fλ bei Laserstrahlen L, Lλ unterschiedlicher Wellenlänge verschieden, wie in Figur 6 schematisch dargestellt ist. Da neben der Verschiebung des Fokus respektive der Abbildungsflächen F, Fλ auch die Fokussierung nicht optimal ist, werden für verschiedene Wellenlängen unterschiedliche Zoomfunktionen vorgesehen. Wie in der Figur 6 ersichtlich ist, wird durch die Wahl eines kurwelligeren Laserstrahls Lλ die Spotgrösse im Vergleich zu der Spotgrösse eines langwelligeren Laserstrahls L reduziert, d.h. der Durchmesser dλ des Spots Sλ des kurzwelligeren Laserstrahls Lλ ist kleiner als der Durchmesser d des Spots S des langwelligeren Laserstrahls L, dλ<d. Eine kleinere Spotgrösse ermöglicht feinere und präzisere Gewebeschnitte mit geringerer Gasbildung und reduzierter Beeinträchtigung (Schädigung) von umgebendem Gewebe. In der Figur 6 bezeichnen die Bezugszeichen Sz und Sλz die schematisch dargestellte Ausdehnung der Spots S, Sλ entlang der Projektionsrichtung z, wobei das Ausmass (d.h. die Länge) dieser Spotausdehnung Sz, Sλz in der Projektionsrichtung z nicht bloss von der Wellenlänge des Laserstrahls L, Lλ sondern auch von der numerischen Apertur NA der Projektionsoptik 11 abhängt: Sz, Sλz ∝ λ/NA². Die Spotausdehnung Sz, Sλz in der Projektionsrichtung z wird in ausgewählten Betriebsmodi für die Erzeugung von Gewebeschnitten in der Projektionsrichtung z (vertikale Schnitte) genutzt, um mit weniger Pulsen effizienter, also schneller, zu schneiden, beispielsweise im nachfolgend beschriebenen ersten Betriebsmodus zum Erzeugen von vertikalen Gewebeschnitten in der Augenlinse 21, um diese zu segmentieren, oder im nachfolgend beschriebenen fünften Betriebsmodus zum Erzeugen von vertikalen Gewebeschnitten in der Hornhaut 22, um diese für eine teilweise oder vollständige Hornhauttransplantation lamellar respektive penetrierend zu schneiden.

Das Steuersystem 100 selektiert die Wellenlänge des gepulsten Laserstrahls L und die auszuführende Zoomfunktion 101, 102, 103, 104, 105 mit zugeordneter Abbildungsfläche F, F1, F2 (Behandlungstiefe), Spotgrössed und Intensitätsprofil, jeweils abhängig von einem gewählten Betriebsmodus. Abhängig von Ausführungsvariante und Konfiguration sind beispielsweise die nachfolgend beschriebenen Betriebsmodi wähl- und ausführbar. Der aktuelle Betriebsmodus wird beispielsweise vom Benutzer über eine Benutzerschnittstelle selektiert und aktiviert oder automatisch durch das Steuersystem 100 abhängig von einem detektierten Vorrichtungstyp des Applikationskopfs 14, der Projektionsoptik 11 und/oder des Kontaktkörpers 110 selektiert und aktiviert, welche aktuell mit der ophthalmologischen Vorrichtung 1 verbunden sind. Der Vorrichtungstyp ist beispielsweise eine mechanische, elektrische, elektronische, optische und/oder elektromagnetische Kennung, die am Applikationskopf 14, an der Projektionsoptik 11 und/oder am/im Kontaktkörper 110 angebracht ist und von einem entsprechenden Detektor der ophthalmologischen Vorrichtung 1 detektiert und an das Steuersystem 100 übermittelt wird.

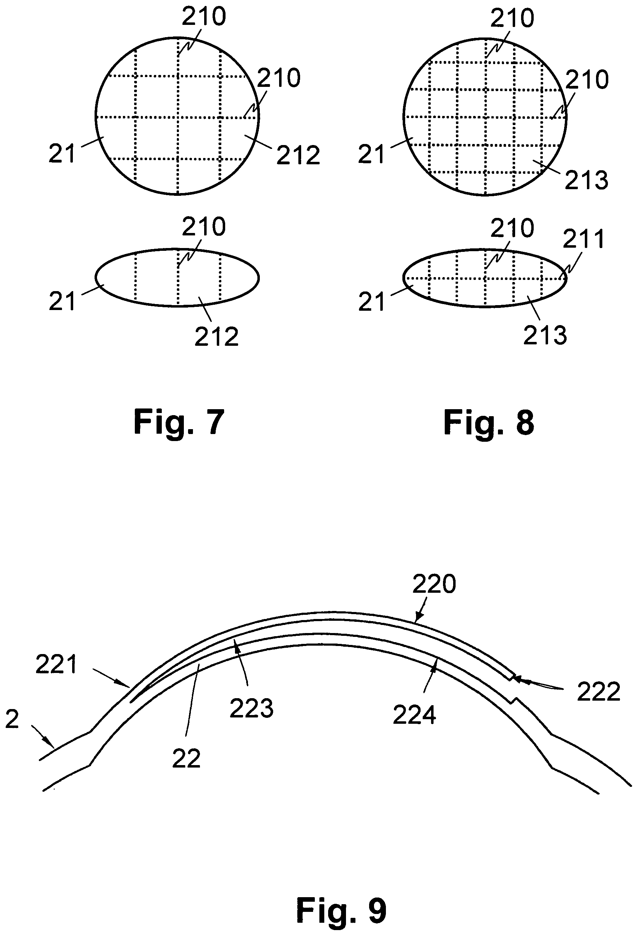

Ein erster Betriebsmodus ist vorgesehen zum Auflösen von Augengewebe der Augenlinse 21 durch Fokussieren von gepulsten Laserstrahlen L im IR-A-Infrarotbereich mit einer ersten Zoomfunktion 101 auf die Abbildungsfläche F1 mit einem Spot S1, der eine Spotgrösse mit einem Durchmesser d 1 aufweist (siehe Figur 1). Wie in den Figuren 7 und 8 jeweils in der Aufsicht (oben) und im Querschnitt (unten) schematisch dargestellt ist, wird der erste Betriebsmodus beispielsweise für die Fragmentierung der Linse 21 verwendet. Im Beispiel der Figur 7, wird die Linse 21 durch vertikale, in Projektionsrichtung z verlaufende Schnitte 210 in Segmente 212 zerteilt, im einfachsten Fall mittels zwei sich im Zentrum der Linse 21 kreuzenden vertikalen Schnitten 210. Im Beispiel der Figur 8 wird überdies auch mindestens ein horizontaler, normal zur Projektionsrichtung z verlaufender Schnitt 211 ausgeführt um die Linse 21 in kleinere Volumenelemente zu zerlegen ("slice and dice"). Das Auflösen von Augengewebe ist insbesondere interessant bei Kataraktoperationen zur Entfernung des Linsenkerns. Grosse Strahltaillen in Kombination mit grossen Pulsenergien sind besonders effizient beim schnellen Auflösen von Linsengewebe. IR-A ist aufgrund der geringen intraokularen Absorption die bevorzugte Wellenlänge für Anwendungen in der Linse 21.

Ein zweiter Betriebsmodus ist vorgesehen zum Erzeugen von horizontalen (d.h. im Wesentlichen parallel zu einer auf die Hornhaut 22, z.B. mittels eines Kontaktkörpers 110, applizierten Referenzfläche verlaufenden) Gewebeschnitten s in der Hornhaut 22 durch Fokussieren der gepulsten Laserstrahlen L im UVA-Ultraviolettbereich mit einer zweiten Zoomfunktion 102 auf die Abbildungsfläche F2 mit einem Spot S2, der eine Spotgrösse mit einem Durchmesser d2 aufweist (siehe Figur 1). Durch den Einsatz von UVA und angepassten Zoomfunktionen lassen sich kleine Strahltaillen erzeugen. Kleine Strahltaillen in Kombination mit kleinen (bei kleinen Strahltaillen ausreichenden) Pulsenergien ermöglichen Schnitte mit optischer Oberflächengüte und präzise Schnittgeometrien. Im Vergleich dazu würde die Verwendung von IRA mit grossen Strahltaillen, die für Anwendungen in der Linse 21 ideal sind, aufgrund der grossen Strahltaillen zu optisch rauen Oberflächen und aufgrund der grossen Pulsenergien zu starker Gasentwicklung mit nachfolgender Deformation der Schnittoberflächen führen. Die ist für Anwendungen im Bereich der optischen Zone der Hornhaut 22 nicht erwünscht.

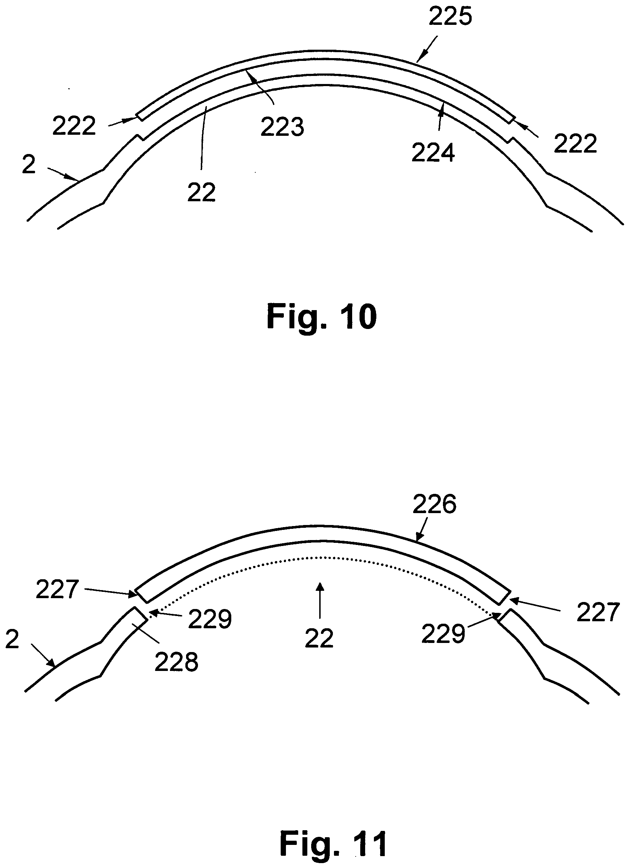

Wie in den Figuren 9 und 10 jeweils im Querschnitt dargestellt ist, wird der zweite Betriebsmodus beispielsweise zum Schneiden von Gewebestücken in der Hornhaut 22 verwendet. Im Beispiel der Figur 9 wird im applanierten Zustand der Hornhaut 22 mittels eines horizontalen, parallel zur Kontaktkörperoberfläche (respektive normal zur Projektionsrichtung z) verlaufenden Schnitts 224 im Innern der Hornhaut 22 ein Gewebelappen 220 ("flap") erzeugt, z.B. für eine LASIK-Behandlung, der in einem Restbereich 221 mit dem Auge 2 verbunden bleibt. Der Gewebelappen 220 weist Seitenflächen 222 auf, die im applanierten Zustand der Hornhaut 22 durch einen vertikalen, in Projektionsrichtung z verlaufenden Schnitt erzeugt werden. Im Beispiel der Figur 10 wird, z.B. für eine lamellare Hornhauttransplantation, im applanierten Zustand der Hornhaut 22 mittels eines horizontalen, normal zur Projektionsrichtung z verlaufenden Schnitts 224 im Innern der Hornhaut 22 ein Teilstück 225 der Hornhaut 22 weggeschnitten, das umlaufend durch Seitenflächen 222 begrenzt ist, die im applanierten Zustand der Hornhaut 22 durch einen umlaufenden vertikalen, in Projektionsrichtung z verlaufenden Schnitt erzeugt werden.

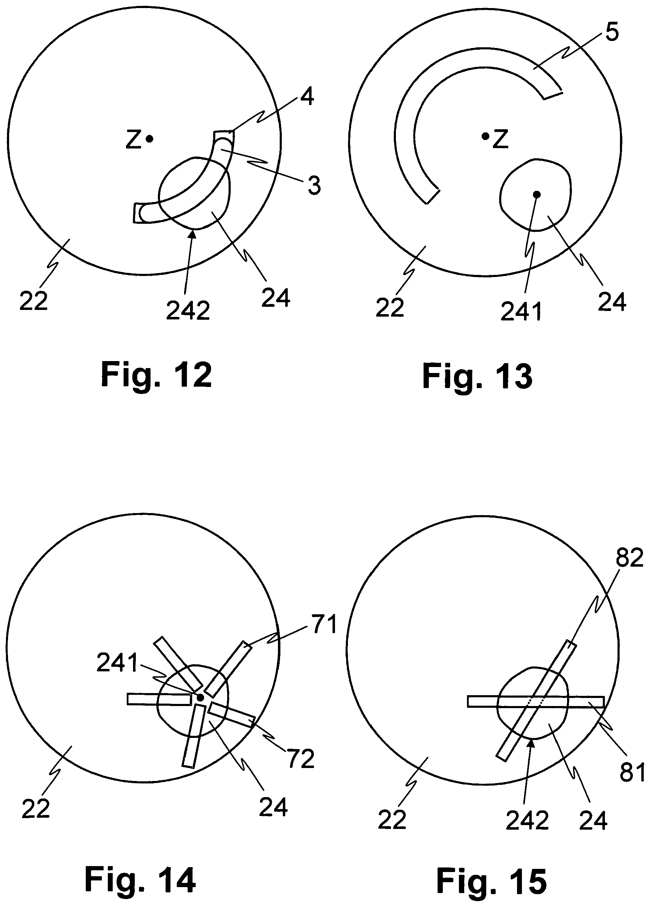

Der zweite Betriebsmodus wird überdies verwendet zum Schneiden von Taschen ("pockets"), von Lentikeln mittels mindestens zwei gekrümmten, sich schneidenden Flächen, und von lamellaren Schnitten in der Hornhaut 22. Wie in den Figuren 12 bis 15 jeweils in der Aufsicht auf die Hornhaut 22 dargestellt ist, wird der zweite Betriebsmodus beispielsweise bei der Behandlung eines Keratokonus 24, d.h. einer krankhaften Verdünnung und Ausstülpung der Hornhaut 22 (Hornhautverdünnung), zum Schneiden von Taschen in der Hornhaut 22 verwendet. Die Figur 12 illustriert die Stützung des Keratokonus 24 mittels eines Implantats 3 (mechanische Stützeinlage), das in eine in die Hornhaut 22 geschnittene Tasche 4 eingeführt wird, z.B. ein so genanntes Intac aus Kunststoff. Im Beispiel der Figur 12 wird die Tasche 4 zum Keratokonusrand 242 zentriert angeordnet geschnitten, wobei die Krümmung der Tasche 4 beispielsweise durch einen Kreisbogen um das Pupillenzentrum Z definiert ist.

Die Figur 13 illustriert das Glattziehen der durch das Keratokonus 24 erzeugten Ausbeulung der Hornhaut 22 mittels eines nicht gezeigten Implantats, das in eine in die Hornhaut 22 geschnittene Tasche 5 eingeführt wird. Im Beispiel der Figur 13 wird die Tasche 5 auf der dem Keratokonuszentrum 241 gegenüberliegenden Seite des Pupillenzentrums Z angeordnet geschnitten, wobei die Krümmung der Tasche 5 beispielsweise durch einen Kreisbogen um das Pupillenzentrum Z definiert ist.

Die Figur 14 illustriert die Korrektur des Keratokonus 24, durch Schneiden mehrerer Taschen 71, 72 in der Hornhaut 22. Im Beispiel der Figur 14 werden die Taschen 71, 72 sternförmig zum Keratokonuszentrum 241 angeordnet geschnitten, wobei die Einstiche respektive Öffnungen der Taschen 71, 72 jeweils auf der vom Keratokonuszentrum 241 abgewandten Seite der Taschen 71, 72 geschnitten werden.

Die Figur 15 illustriert die Stützung des Keratokonus 24 mittels mechanischen Stützeinlagen, welche in Taschen 81, 82 eingeführt werden, die mit einem Abstand übereinanderliegend und sich kreuzend in die Hornhaut 22 geschnitten werden. Im Beispiel der Figur 15 wird die durch die Schnittflächen definierte Kreuzform zum Keratokonusrand 242 zentriert angeordnet.

Alternativ zum Einbringen von stabilisierenden Implantaten lassen sich auch Klebstoffe einsetzen. Hier ist insbesondere Riboflavin zu nennen, das sich durch die Bestrahlung mit UVA aktivieren lässt. Soll die Aktivierung des vom Gewebe aufgenommenen Riboflavins mit dem Laserstrahl L erfolgen, dann kann mit dem Fokussiersystem 10 eine breite und gestreckte Strahltaille mit eingestellt werden. Somit ist es möglich, grosse Gewebebereiche schneller zu belichten als mit einem scharf fokussierten Strahl.

Ein dritter Betriebsmodus ist vorgesehen zum Auflösen von Augengewebe der Augenlederhaut 23 oder getrübten Hornhaut 22 durch Fokussieren von gepulsten Laserstrahlen L im IR-B-Infrarotbereich mit einer dritten Zoomfunktion 103, z.B. auf die Abbildungsfläche F3 mit einem Spot S3, der eine Spotgrösse mit einem Durchmesser d3 aufweist (siehe Figur 1).

Ein vierter Betriebsmodus ist vorgesehen zum Erzeugen von Gewebeschnitten im vorderen Linsensack der Augenlinse 21 durch Fokussieren der gepulsten Laserstrahlen L im UVA-Ultraviolettbereich mit einer vierten Zoomfunktion 104. Im vierten Betriebsmodus wird beispielsweise mittels eines vertikalen, in der Projektionsrichtung z verlaufenden, umlaufenden Schnitts eine zylinderförmige Öffnung in den vorderen Linsensack der Augenlinse 21 geschnitten, durch welchen die segmentierte oder fragmentierte Augenlinse 21 entfernt und mit einer künstlichen Linse ersetzt werden kann.

Ein fünfter Betriebsmodus ist vorgesehen zum Erzeugen von vertikalen (d.h. im Wesentlichen normal zu einer auf die Hornhaut, z.B. mittels eines Kontaktkörpers 110, applizierten Referenzfläche verlaufenden) Gewebeschnitten in der Hornhaut 22 durch Fokussieren von gepulsten Laserstrahlen L im IR-A-Infrarotbereich mit einer fünften Zoomfunktion 105. Wie in der Figur 11 im Querschnitt dargestellt ist, wird der fünfte Betriebsmodus beispielsweise zum Wegschneiden der Hornhaut 22 für eine vollständige Hornhauttransplantation verwendet. Im Beispiel der Figur 11 wird im applanierten Zustand der Hornhaut 22 mittels eines vertikalen, in der Projektionsrichtung z verlaufenden, umlaufenden Schnitts 229 die Hornhaut 22 penetrierend durchschnitten und mindestens ein Hornhautteilstück 226 vom Auge 2 getrennt. Da bei dieser Anwendung die Schnitte ausserhalb der optischen Zone des Auges 2 liegen, stören optisch raue Oberflächen nicht. Auch sind Deformationen (typisch 2-20 µm) des Gewebes durch starke Gasentwicklung infolge höherer Pulsenergien unkritisch. Im Gegenteil, durch breitere, gestreckte Strahltaillen lässt es sich schneller schneiden und eine vollständige Gewebetrennung erreichen.

## Patentansprüche

1. Ophthalmologische Vorrichtung (1) zur Behandlung von Augengewebe mittels gepulster Laserstrahlen (L), umfassend ein Lasersystem (12), das eingerichtet ist, in einem ersten Betriebsmodus gepulste Laserstrahlen (L) mit einer Wellenlänge im NIR-Infrarotbereich zu erzeugen und in einem zweiten Betriebsmodus gepulste Laserstrahlen (L) mit einer Wellenlänge im UVA-Ultraviolettbereich zu erzeugen,
**gekennzeichnet durch** ein Fokussiersystem (10) mit einer Projektionsoptik (11), das eingerichtet ist,
im ersten Betriebsmodus die gepulsten Laserstrahlen (L) im NIR-Infrarotbereich mit einer ersten Zoomfunktion (101) zum Auflösen von Augengewebe auf eine erste Spotgrösse (d1) fokussiert in die Augenlinse (21) zu projizieren, und
im zweiten Betriebsmodus die gepulsten Laserstrahlen (L) im UVA-Ultraviolettbereich mit einer von der ersten Zoomfunktion (101) verschiedenen, zweiten Zoomfunktion (102) zum Erzeugen von Gewebeschnitten (s) auf eine zweite Spotgrösse (d2), die wesentlich kleiner als die erste Spotgrösse (d1) ist, fokussiert in die Augenhornhaut (22) zu projizieren.

2. Vorrichtung (1) nach Anspruch 1, **dadurch gekennzeichnet, dass** das Fokussiersystem (10) eingerichtet ist, im ersten Betriebsmodus die gepulsten Laserstrahlen (L) im NIR-Infrarotbereich mit der ersten Zoomfunktion (101) auf eine in der Augenlinse (21) liegende Abbildungsfläche (F1) zu projizieren und auf die erste Spotgrösse (d1) zu fokussieren, und im zweiten Betriebsmodus die gepulsten Laserstrahlen (L) im UVA-Ultraviolettbereich mit der zweiten Zoomfunktion (102) auf eine in der Augenhornhaut (22) liegende Abbildungsfläche (F2) zu projizieren und auf die zweite Spotgrösse (d2) zu fokussieren.

3. Vorrichtung (1) nach einem der Ansprüche 1 oder 2, **dadurch gekennzeichnet, dass** das Fokussiersystem (10) zwei Optiksysteme (111, 112) umfasst, welche durch die Zoomfunktionen (101, 102) einstellbar sind, die gepulsten Laserstrahlen (L) auf eine Abbildungsfläche (F1, F2) zu projizieren und auf der Abbildungsfläche (F1, F2) auf eine Spotgrösse (d1, d2) zu fokussieren.

4. Vorrichtung (1) nach Anspruch 3, **dadurch gekennzeichnet, dass** das Fokussiersystem (10) ein durch die Zoomfunktionen (101, 102) steuerbares Antriebssystem (106) zum individuellen Einstellen der Optiksysteme (111, 112) umfasst.

5. Vorrichtung (1) nach einem der Ansprüche 3 oder 4, **dadurch gekennzeichnet, dass** das Fokussiersystem (10) eingerichtet ist, im ersten Betriebsmodus mit der ersten Zoomfunktion (101) die Optiksysteme (111, 112) einzustellen, die gepulsten Laserstrahlen (L) im NIR-Infrarotbereich auf die in der Augenlinse (21) liegende Abbildungsfläche (F1) zu projizieren und auf der in der Augenlinse (21) liegenden Abbildungsfläche (F1) auf die erste Spotgrösse (d1) zu fokussieren, und dass das Fokussiersystem (10) eingerichtet ist, im zweiten Betriebsmodus mit der zweiten Zoomfunktion (102) die Optiksysteme (111, 112) einzustellen, die gepulsten Laserstrahlen (L) im UVA-Ultraviolettbereich auf die in der Augenhornhaut (22) liegende Abbildungsfläche (F2) zu projizieren und auf der in der Augenhornhaut (22) liegenden Abbildungsfläche (F2) auf die zweite Spotgrösse (d2) zu fokussieren.

6. Vorrichtung (1) nach einem der Ansprüche 1 bis 5, **dadurch gekennzeichnet, dass** das Fokussiersystem (10) mindestens eines umfasst aus: in den Strahlengang einschiebbare optische Linsen, deformierbare Spiegel, mechanisch ausgeführte Zoomkurven zur Ausführung der Zoomfunktionen (101, 102, 103, 104), und Zoomfunktionen (101, 102, 103, 104) mit digitalisierten Zoomkurven für ein Steuersystem (100).

7. Vorrichtung (1) nach einem der Ansprüche 1 bis 6, **dadurch gekennzeichnet, dass** das Lasersystem (12) eingerichtet ist, im ersten Betriebsmodus gepulste Laserstrahlen (L) mit einer Wellenlänge im IR-A-Infrarotbereich zu erzeugen und in einem dritten Betriebsmodus gepulste Laserstrahlen (L) mit einer Wellenlänge im IR-B-Infrarotbereich zu erzeugen, und dass das Fokussiersystem (10) eingerichtet ist, im dritten Betriebsmodus die gepulsten Laserstrahlen (L) im IR-B-Infrarotbereich zum Auflösen von Augengewebe fokussiert in die Augenlederhaut (23) oder getrübte Hornhaut (22) zu projizieren.

8. Vorrichtung (1) nach Anspruch 7, **dadurch gekennzeichnet, dass** das Fokussiersystem (10) eingerichtet ist, im dritten Betriebsmodus die gepulsten Laserstrahlen (L) im IR-B-Infrarotbereich mit einer dritten Zoomfunktion (103) auf eine in der Augenlederhaut (23) oder einer getrübten Hornhaut (22) liegende Abbildungsfläche (F3) zu projizieren und auf eine dritte Spotgrösse (d3) zu fokussieren.

9. Vorrichtung (1) nach einem der Ansprüche 1 bis 8, **dadurch gekennzeichnet, dass** das Lasersystem (12) eingerichtet ist, in verschiedenen Betriebsmodi gepulste Laserstrahlen (L) mit unterschiedlichen Wellenlängen zu erzeugen, und dass das Fokussiersystem (10) eingerichtet ist, in den verschiedenen Betriebsmodi die gepulsten Laserstrahlen (L) mit den verschiedenen Wellenlängen jeweils mit einer der betreffenden Wellenlänge zugeordneten unterschiedlichen Zoomfunktion (101, 102, 103, 104) fokussiert in das Augengewebe zu projizieren.

10. Vorrichtung (1) nach einem der Ansprüche 1 bis 9, **dadurch gekennzeichnet, dass** das Fokussiersystem (10) eingerichtet ist, die gepulsten Laserstrahlen (L) in verschiedenen Betriebsmodi mit einem unterschiedlichen, durch eine dem betreffenden Betriebsmodus zugeordnete Zoomfunktion (101, 102, 103, 104) bestimmten Intensitätsprofil fokussiert auf die Abbildungsfläche (F, F1, F2, F3) zu projizieren.

11. Vorrichtung (1) nach einem der Ansprüche 1 bis 10, **gekennzeichnet durch** ein Steuersystem (100), das eingerichtet ist, die Vorrichtung (1) gemäss verschiedenen Betriebsmodi zu steuern, wobei die Betriebsmodi mindestens eines umfassen aus:
- einen ersten Betriebsmodus zum Auflösen von Augengewebe der Augenlinse (21) durch Fokussieren von gepulsten Laserstrahlen (L) im IR-A-Infrarotbereich mit einer ersten Zoomfunktion (101),
- einen zweiten Betriebsmodus zum Erzeugen von horizontalen Gewebeschnitten (s) in der Hornhaut (22) durch Fokussieren der gepulsten Laserstrahlen (L) im UVA-Ultraviolettbereich mit einer zweiten Zoomfunktion (102),
- einen dritten Betriebsmodus zum Auflösen von Augengewebe der Augenlederhaut (23) oder getrübten Hornhaut (22) durch Fokussieren von gepulsten Laserstrahlen (L) im IR-B-Infrarotbereich mit einer dritten Zoomfunktion (103),
- einen vierten Betriebsmodus zum Erzeugen von Gewebeschnitten im Linsensack der Augenlinse (21) durch Fokussieren der gepulsten Laserstrahlen (L) im UVA-Ultraviolettbereich mit einer vierten Zoomfunktion (104), und
- einen fünften Betriebsmodus zum Erzeugen von vertikalen Gewebeschnitten in der Hornhaut (22) durch Fokussieren von gepulsten Laserstrahlen (L) im IR-A-Infrarotbereich mit einer fünften Zoomfunktion (105).

12. Vorrichtung (1) nach einem der Ansprüche 1 bis 11, **dadurch gekennzeichnet, dass** die Projektionsoptik (11) für die gepulsten Laserstrahlen (L) im UVA-Ultraviolettbereich transparent ausgeführt ist und eine tiefe numerische Apertur NA, insbesondere eine numerische Apertur NA<0.5, aufweist.

13. Vorrichtung (1) nach einem der Ansprüche 1 bis 12, **dadurch gekennzeichnet, dass** die Projektionsoptik (11) eine numerische Apertur NA<0.3 aufweist.

14. Vorrichtung (1) nach einem der Ansprüche 1 bis 12, **dadurch gekennzeichnet, dass** die Projektionsoptik (11) eine numerische Apertur NA<0.2 aufweist.

15. Vorrichtung (1) nach einem der Ansprüche 1 bis 14, **dadurch gekennzeichnet, dass** in der Projektionsoptik (11) verwendete Gläser einen Brechungsindex n<1.65 aufweisen und insbesondere aus Quarzglas gefertigt sind.

## Claims

1. An ophthalmological device (1) for treating eye tissue by means of pulsed laser beams (L), comprising a laser system (12) which is designed, in a first mode of operation, to generate pulsed laser beams (L) with a wavelength in the NIR infrared range and, in a second mode of operation, to generate pulsed laser beams (L) with a wavelength in the UVA ultraviolet range,
**characterized by** a focusing system (10) with a projection optical unit (11), which is designed,
in the first mode of operation, to project the pulsed laser beams (L) in the NIR infrared range into the lens (21) of the eye, which pulsed laser beams are focused to a first spot size (d1) by means of a first zoom function (101) for the purpose of disintegrating eye tissue, and,
in the second mode of operation, to project the pulsed laser beams (L) in the UVA ultraviolet range into the cornea (22) of the eye, which pulsed laser beams are focused to a second spot size (d2) which is substantially smaller than the first spot size (d1) by means of a second zoom function (102), which differs from the first zoom function (101), for the purpose of creating tissue cuts (s).

2. The device (1) as claimed in claim 1, wherein the focusing system (10) is designed, in the first mode of operation and by using the first zoom function (101), to project the pulsed laser beams (L) in the NIR infrared range onto an imaging surface (F1) situated in the lens (21) of the eye and to focus said pulsed laser beams onto the first spot size (d1) and, in the second mode of operation and by using the second zoom function (102), to project the pulsed laser beams (L) in the UVA ultraviolet range onto an imaging surface (F2) situated in the cornea (22) of the eye and to focus said pulsed laser beams onto the second spot size (d2).

3. The device (1) as claimed in either of claims 1 or 2, wherein the focusing system (10) comprises two optical systems (111,112), which can be set by the zoom functions (101, 102) to project the pulsed laser beams (L) onto an imaging surface (F1, F2) and to focus said laser beams to a spot size (d1, d2) on the imaging surface (F1, F2).

4. The device (1) as claimed in claim 3, wherein the focusing system (10) comprises a drive system (106), which can be controlled by the zoom functions (101, 102), for individual setting of the optical systems (111, 112).

5. The device (1) as claimed in either of claims 3 or 4, wherein the focusing system (10) is designed, in the first mode of operation and by using the first zoom function (101), to set the optical systems (111, 11 2) to project the pulsed laser beams (L) in the NIR infrared range onto the imaging surface (F1) situated in the lens (21) of the eye and, on the imaging surface (F1) situated in the lens (21) of the eye, to focus said pulsed laser beams onto the first spot size (d1) and wherein the focusing system (10) is designed, in the second mode of operation and by using the second zoom function (102), to set the optical systems (111, 112) to project the pulsed laser beams (L) in the UVA ultraviolet range onto the imaging surface (F2) situated in the cornea (22) of the eye and, on the imaging surface (F2) situated in the cornea (22) of the eye, to focus said pulsed laser beams onto the second spot size (d2).

6. The device (1) as claimed in one of claims 1 to 5, wherein the focusing system (10) comprises at least one of the following: optical lenses that can be inserted into the beam path, deformable mirrors, mechanically embodied zoom curves for carrying out the zoom functions (101, 102, 103, 104) and zoom functions (101, 102, 103, 104) with digitized zoom curves for a control system (100).

7. The device (1) as claimed in one of claims 1 to 6, wherein the laser system (12) is designed, in the first mode of operation, to generate pulsed laser beams (L) with a wavelength in the IR-A infrared range and, in a third mode of operation, to generate pulsed laser beams (L) with a wavelength in the IR-B infrared range and wherein the focusing system (10) is designed, in the third mode of operation, to project the pulsed laser beams (L) in the IR-B infrared range in focus into the sclera (23) of the eye or the cloudy cornea (22) for the purpose of disintegrating eye tissue.

8. The device (1) as claimed in claim 7, wherein the focusing system (10) is designed, in the third mode of operation and by using a third zoom function (103), to project the pulsed laser beams (L) in the IR-B infrared range onto an imaging surface (F3) situated in the sclera (23) of the eye or in a cloudy cornea (22) and to focus said pulsed laser beams onto a third spot size (d3).

9. The device (1) as claimed in one of claims 1 to 8, wherein the laser system (12) is designed, in various modes of operation, to generate pulsed laser beams (L) with different wavelengths and wherein the focusing system (10) is designed, in the various modes of operation, to project the pulsed laser beams (L) with the different wavelengths into the eye tissue, respectively focused by means of a different zoom function (101, 102, 103, 104) associated with the relevant wavelength.

10. The device (1) as claimed in one of claims 1 to 9, wherein the focusing system (10) is designed, in various modes of operation, to project the pulsed laser beams (L) in focus onto the imaging surface (F, F1, F2, F3) using a different intensity profile which is determined by a zoom function (101, 102, 103, 104) associated with the relevant mode of operation.

11. The device (1) as claimed in one of claims 1 to 10, **characterized by** a control system (100) which is designed to control the device (1) in accordance with different modes of operation, wherein the modes of operation comprise at least one of the following:
- a first mode of operation for disintegrating eye tissue of the lens (21) of the eye by focusing pulsed laser beams (L) in the IR-A infrared range by means of a first zoom function (101),
- a second mode of operation for creating horizontal tissue cuts (s) in the cornea (22) by focusing the pulsed laser beams (L) in the UVA ultraviolet range by means of a second zoom function (102),
- a third mode of operation for disintegrating eye tissue of the sclera (23) of the eye or the cloudy cornea (22) by focusing pulsed laser beams (L) in the IR-B infrared range by means of a third zoom function (103),
- a fourth mode of operation for creating tissue cuts in the capsular bag of the lens (21) of the eye by focusing the pulsed laser beams (L) in the UVA ultraviolet range by means of a fourth zoom function (104), and
- a fifth mode of operation for creating vertical tissue cuts in the cornea (22) by focusing pulsed laser beams (L) in the IR-A infrared range by means of a fifth zoom function (105).

12. The device (1) as claimed in one of claims 1 to 11, wherein the projection optical unit (11) for the pulsed laser beams (L) is designed to be transparent in the UVA ultraviolet range and has a low numerical aperture NA, in particular a numerical aperture NA < 0.5.

13. The device (1) as claimed in one of claims 1 to 12, wherein the projection optical unit (11) has a numerical aperture NA < 0.3.

14. The device (1) as claimed in one of claims 1 to 12, wherein the projection optical unit (11) has a numerical aperture NA < 0.2.

15. The device (1) as claimed in one of claims 1 to 14, wherein glasses used in the projection optical unit (11) have a refractive index n < 1.65 and are, in particular, made of fused quartz.

## Revendications

1. Dispositif ophtalmologique (1) destiné au traitement de tissus oculaires au moyen de faisceaux laser à impulsions (L), comprenant un système de laser (12), lequel est configuré en vue de générer des faisceaux laser à impulsions (L) avec une longueur d'onde dans la plage des infrarouges proches (NIR), dans un premier mode de fonctionnement, et en vue de générer des faisceaux laser à impulsions (L) avec une longueur d'onde dans la plage des ultraviolets de type UVA, dans un deuxième mode de fonctionnement,
**caractérisé par** un système de focalisation (10) qui comprend une optique de projection (11) et qui est configuré en vue de :
projeter, dans le premier mode de fonctionnement, de manière concentrée dans le cristallin de l'oeil (21), les faisceaux laser à impulsions (L) dans la plage des infrarouges NIR au moyen d'une première fonction de zoom (101), en vue de l'ablation de tissus oculaires sur une première taille de points (d1) ; et
projeter, dans le deuxième mode de fonctionnement, de manière concentrée dans la cornée de l'oeil (22), les faisceaux laser à impulsions (L) dans la plage des ultraviolets de type UVA au moyen d'une deuxième fonction de zoom (102), laquelle est différente de la première fonction de zoom (101), en vue de la réalisation de coupes de tissu (s) sur une deuxième taille de points (d2), laquelle est sensiblement inférieure à la première taille de points (d1).

2. Dispositif (1) selon la revendication 1, **caractérisé en ce que** le système de focalisation (10) est configuré en vue de projeter, dans le premier mode de fonctionnement, les faisceaux laser à impulsions (L) dans la plage des infrarouges NIR au moyen de la première fonction de zoom (101) sur une surface de formation d'image (F1) qui se trouve dans le cristallin de l'oeil (21) et en vue de réaliser une focalisation sur la première taille de points (d1), d'une part, et en vue de projeter, dans le deuxième mode de fonctionnement, les faisceaux laser à impulsions (L) dans la plage des ultraviolets de type UVA au moyen de la deuxième fonction de zoom (102) sur une surface de formation d'image (F2) qui se trouve dans la cornée de l'oeil (22) et en vue de réaliser une focalisation sur la deuxième taille de points (d2), d'autre part.

3. Dispositif (1) selon l'une des revendications 1 ou 2, **caractérisé en ce que** le système de focalisation (10) comprend deux systèmes optiques (111, 112), lesquels peuvent être réglés par l'intermédiaire des fonctions de zoom (101, 102), en vue de projeter les faisceaux laser à impulsions (L) sur une surface de formation d'image (F1, F2) et en vue de réaliser une focalisation sur une taille de points (d1, d2) sur la surface de formation d'image (F1, F2).

4. Dispositif (1) selon la revendication 3, **caractérisé en ce que** le système de focalisation (10) comprend un système d'entraînement (106) pouvant être commandé par l'intermédiaire des fonctions de zoom (101, 102) en vue du réglage individuel des systèmes optiques (111, 112).

5. Dispositif (1) selon l'une des revendications 3 ou 4, **caractérisé en ce que** le système de focalisation (10) est configuré en vue de régler les systèmes optiques (111, 112) au moyen de la première fonction de zoom (101), dans le premier mode de fonctionnement, en vue de projeter les faisceaux laser à impulsions (L) dans la plage des infrarouges NIR sur la surface de formation d'image (F1) qui se trouve dans le cristallin de l'oeil (21) et en vue de réaliser une focalisation sur la première taille de points (d1) sur la surface de formation d'image (F1) qui se trouve dans le cristallin de l'oeil (21), et **caractérisé en ce que** le système de focalisation (10) est configuré en vue de régler les systèmes optiques (111, 112) au moyen de la deuxième fonction de zoom (102), dans le deuxième mode de fonctionnement, en vue de projeter les faisceaux laser à impulsions (L) dans la plage des ultraviolets de type UVA sur la surface de formation d'image (F2) qui se trouve dans la cornée de l'oeil (22) et en vue de réaliser une focalisation sur la deuxième taille de points (d2) sur la surface de formation d'image (F2) qui se trouve dans la cornée de l'oeil (22).

6. Dispositif (1) selon l'une des revendications 1 à 5, **caractérisé en ce que** le système de focalisation (10) comprend au moins l'un des éléments suivants : des lentilles optiques pouvant être glissées dans la trajectoire du faisceau, des miroirs déformables, des courbes de zoom réalisées de manière mécanique en vue de la réalisation des fonctions de zoom (101, 102, 103, 104), ainsi que des fonctions de zoom (101, 102, 103, 104) avec des courbes de zoom numérisées pour un système de commande (100).

7. Dispositif (1) selon l'une des revendications 1 à 6, **caractérisé en ce que** le système de laser (12) est configuré en vue de générer des faisceaux laser à impulsions (L) avec une longueur d'onde dans la plage des infrarouges de type A, dans le premier mode de fonctionnement, et en vue de générer des faisceaux laser à impulsions (L) avec une longueur d'onde dans la plage des infrarouges de type B, dans un troisième mode de fonctionnement, et **caractérisé en ce que** le système de focalisation (10) est configuré en vue de projeter, de manière concentrée dans la sclère de l'oeil (23) ou dans la cornée (22) opacifiée, les faisceaux laser à impulsions (L) dans la plage des infrarouges de type B en vue de l'ablation de tissus oculaires, dans le troisième mode de fonctionnement.

8. Dispositif (1) selon la revendication 7, **caractérisé en ce que** le système de focalisation (10) est configuré en vue de projeter les faisceaux laser à impulsions (L) dans la plage des infrarouges de type B au moyen d'une troisième fonction de zoom (103) sur une surface de formation d'image (F3) qui se trouve dans la sclère de l'oeil (23) ou d'une cornée (22) opacifiée, dans le troisième mode de fonctionnement, et en vue de réaliser une focalisation sur une troisième taille de points (d3).

9. Dispositif (1) selon l'une des revendications 1 à 8, **caractérisé en ce que** le système de laser (12) est configuré en vue de générer des faisceaux laser à impulsions (L) avec diverses longueurs d'onde, dans différents modes de fonctionnement, et **caractérisé en ce que** le système de focalisation (10) est configuré en vue de projeter de manière concentrée dans le tissu oculaire, les faisceaux laser à impulsions (L) avec les diverses longueurs d'onde, dans les différents modes de fonctionnement, respectivement au moyen d'une fonction de zoom (101, 102, 103, 104) différente qui est associée à la longueur d'onde concernée.

10. Dispositif (1) selon l'une des revendications 1 à 9, **caractérisé en ce que** le système de focalisation (10) est configuré en vue de projeter, de manière concentrée sur la surface de formation d'image (F, F1, F2, F3), les faisceaux laser à impulsions (L) avec un profil d'intensité différent qui est déterminé par une fonction de zoom (101, 102, 103, 104) associée au mode de fonctionnement concerné, dans différents modes de fonctionnement.

11. Dispositif (1) selon l'une des revendications 1 à 10, **caractérisé par** un système de commande (100), lequel est configuré en vue de piloter le dispositif (1) en fonction de différents modes de fonctionnement ;
selon lequel les modes de fonctionnement comprennent au moins l'un des modes suivants :
- un premier mode de fonctionnement en vue de l'ablation de tissus oculaires du cristallin de l'oeil (21) par la focalisation des faisceaux laser à impulsions (L) dans la plage des infrarouges de type A, au moyen d'une première fonction de zoom (101) ;
- un deuxième mode de fonctionnement en vue de la réalisation de coupes de tissu (s) horizontales dans la cornée (22) par la focalisation des faisceaux laser à impulsions (L) dans la plage des ultraviolets de type UVA, au moyen d'une deuxième fonction de zoom (102) ;
- un troisième mode de fonctionnement en vue de l'ablation de tissus oculaires de la sclère de l'oeil (23) ou de la cornée (22) opacifiée par la focalisation des faisceaux laser à impulsions (L) dans la plage des infrarouges de type B, au moyen d'une troisième fonction de zoom (103) ;
- un quatrième mode de fonctionnement en vue de la réalisation de coupes de tissu dans le sac capsulaire du cristallin de l'oeil (21) par la focalisation des faisceaux laser à impulsions (L) dans la plage des ultraviolets de type UVA, au moyen d'une quatrième fonction de zoom (104) ; et
- un cinquième mode de fonctionnement en vue de la réalisation de coupes de tissu verticales dans la cornée (22) par la focalisation des faisceaux laser à impulsions (L) dans la plage des infrarouges de type A, au moyen d'une cinquième fonction de zoom (105).

12. Dispositif (1) selon l'une des revendications 1 à 11, **caractérisé en ce que** l'optique de projection (11) est réalisée de manière transparente pour les faisceaux laser à impulsions (L) dans la plage des ultraviolets de type UVA et présente une profonde ouverture numérique NA, en particulier une ouverture numérique où NA < 0,5.

13. Dispositif (1) selon l'une des revendications 1 à 12, **caractérisé en ce que** l'optique de projection (11) présente une ouverture numérique où NA < 0,3.

14. Dispositif (1) selon l'une des revendications 1 à 12, **caractérisé en ce que** l'optique de projection (11) présente une ouverture numérique où NA < 0,2.

15. Dispositif (1) selon l'une des revendications 1 à 14, **caractérisé en ce que** les verres utilisés dans l'optique de projection (11) présentent un indice de réfraction n < 1,65 et sont, en particulier, fabriqués en verre de quartz.
